# EUROPÄISCHE PATENTANMELDUNG

(11) **EP 1 479 391 A1**
(43) Veröffentlichungstag der Anmeldung: **24.11.2004**
(21) Anmeldenummer: 04011924.0
(22) Anmeldetag: 19.05.2004
(51) Int. Cl.: A61K 35/34, C12N 5/06

(54) **Ex-vivo gehaltene, vitale Mammalia-Herzgewebe-Zellen, Verfahren zu deren Gewinnung und Kultivierung und deren Verwendung**

(30) Priorität: 21.05.2003 DE 10322986
(71) Anmelder: KeyNeurotek AG, 39120 Magdeburg (DE)
(72) Erfinder: Götte, Andreas Dr., 37154 Northeim (DE); Röhnert, Peter Dr., 39112 Magdeburg (DE); Lendeckel, Uwe Dr., 39128 Magdeburg (DE); Striggow, Frank Dr., 39175 Gerwisch (DE)
(74) Vertreter: Koepe, Gerd L., Dipl.-Chem.

(57) **Zusammenfassung**

Die Erfindung betrifft ex-vivo unter Simulation physiologischer Bedingungen gehaltene, vitale Mammalia-Herzgewebe-Zellen, insbesondere ex-vivo gehaltene, vitale Mammalia-Herzgewebe-Zellen in üblichem Kulturmedium unter Beaufschlagung mit Kulturgas bei physiologisch annehmbarem pH-Wert, Verfahren zu deren Gewinnung und zu deren Kultivierung sowie deren Verwendung in Modell-Untersuchungen chemischer, biologischer und/oder physikalischer Einflüsse auf physiologische oder pathophysiologische Prozesse.

## Beschreibung

Die Erfindung betrifft ex-vivo gehaltene, vitale Mammalia-Herzgewebe-Zellen, Verfahren zu deren Herstellung und deren Verwendung. Insbesondere betrifft die Erfindung vitale humane oder tierische Herzgewebe-Zellen, die ex vivo gehalten werden, ein Verfahren zur Gewinnung und zur Kultivierung von funktionell intaktem humanem oder tierischem Herzgewebe sowie dessen Nutzung für Untersuchungen zum Einfluß verschiedenster chemischer, biologischer und physikalischer Parameter, wie z.B. elektrische Stimulation, Ischämie, Hypoxie, molekularbiologiche Manipulation, Applikation biologisch aktiver Substanzen, auf physiologische und pathophysiologische Prozesse. Dabei können neben subzellulären und zellulären Veränderungen insbesondere auch Veränderungen im intakten Gewebeverband untersucht und bestimmt werden.

Herzmuskelgewebe besteht zum überwiegenden Teil aus einem dichten Verband von Myozyten, Fibroblasten und Endothelzellen. Mehrere Studien haben gezeigt, dass die Interaktion der Zellen bzw. der Zellpopulationen untereinander grosse Bedeutung für die molekulare Regulation verschiedener Signalprozesse besitzt. So ist zum Beispiel der durch Angiotensin II vermittelte Effekt auf eine parakrine Wirkung zurückzuführen. Auch die Wirkung verschiedener pharmakologischer Substanzen, wie zum Beispiel Antiarrhythmika, scheint von der Zusammensetzung des myokardialen Gewebeverbandes beeinflusst zu sein. Aus diesem Grund ist die Untersuchung von ganzem Gewebe für die Beurteilung von biologischen oder molekularen Prozessen von besonderer Bedeutung und kann eine neue Qualität der Untersuchung darstellen. Bisher war eine solche Untersuchung nur mit großem interventionellem Aufwand und Risiko durch repetitive Myokardbiopsien aus den Ventrikeln bei Patienten bzw. im Ganztierexperiment möglich. Wiederholte Biopsien der Vorhofwand sind *in vivo* sowohl bei Patienten als auch im Tierversuch nicht möglich, da eine Biopsie nahezu immer zur Perforation der Herzwand führt. Sie scheiden daher als regelmäßige Maßnahme zur Gewinnung von beispielsweise humanem Herz-Vorhof-Gewebe praktisch aus. Funktionelle Experimente an solchem Gewebe waren daher bisher vollständig unmöglich.

Die Erfindung hatte daher die Aufgabe, Verfahren zur Gewinnung und Kultivierung von Mammalia-Herzgewebe-Zellen und - im Ergebnis - auch Mammalia-Herzgewebe-Zellen selbst bereitzustellen, wobei die gewonnen und kultivierten Zellen ex vivo gehalten werden können und trotzdem funktionell intakt sind. Die Erfindung hatte weiter die Aufgabe, Verwendungen für solche ex-vivo gehaltenen, vitalen Mammalia-Herzgewebe-Zellen anzugeben.

Der Erfindung liegt der überraschende Befund zugrunde, dass ein von einem Säuger (mammalia), vorzugsweise vom Menschen, entnommener myokardialer Gewebeschnitt bzw. Gewebeblock als eine vitale, intakte funktionale Einheit über mehrere Tage *ex vivo* in Kultur zu halten ist. Damit ermöglicht die vorliegende Erfindung erstmals funktionelle Experimente an Mammalia-Herzgewebe, insbesondere an humanem Vorhof-Gewebe.

Besonders überraschend und unerwartet war die Tatsache, dass nach elektrischer Stimulation der erfindungsgemäß gewonnenen und kultivierten Gewebeschnitte (Simulation von Vorhofflimmern) Veränderungen des atrialen Expressionsmusters auftreten, die zuvor bei Patienten mit chronischem Vorhofflimmern nachgewiesen werden konnten. Darüber hinaus konnte überraschenderweise an elektrisch stimulierten Mammalia-Herzgewebe-Schnitten eine Induktion der mRNA-Expression des κ-Opiat-Rezeptors nachgewiesen werden, wie sie an einem anderen etablierten Modell, den aus Vorläuferzellen *in vitro* differenzierten Kardiomyozyten (P19-Zellen) in gleichem Maße nach elektrischer Stimulation zu beobachten ist.

Daher ermöglicht die vorliegende Erfindung, die Effekte unterschiedlicher chemischer Substanzen (Pharmaka wie zum Beispiel Antiarrhythmika, Rezeptorinhibitoren, natürliche und synthetische Peptide etc.) bzw. Umweltbedingungen (verschiedene Kulturmedien, Sauerstoffentzug, Hypoglykämie, Hyperglykämie, Elektrolytverändrungen, elektrischer Strom etc.) auf die erfindungsgemäß erhaltene myokardiale Gewebekultur zu untersuchen. Ebenfalls erlaubt das Modell Untersuchungen zur Regeneration von durch äußere Einflüsse induzierten Veränderungen des Myokard-Gewebes.

Überraschenderweise ermöglicht die Erfindung weiter - im Gegensatz zu den bisher genutzten Zellkulturen aus isolierten Myozyten - die Untersuchung am intakten Gewebeverband. Die funktionelle Anaylse der Interaktion unterschiedlicher Zellen im Gewebeverband sowohl auf elektrophysiologischer als auch molekularer Ebene kann an den bislang genutzten Kultursystemen aus Myozyten und Fibroblasten nicht untersucht werden, zumal zur Kultivierung von Myozyten häufig auch fetale Zellen benutzt werden, die in ihren Eigenschaften nur bedingt mit adulten humanen Myozyten vergleichbar sind.

Die Erfindung betrifft somit in einem ersten Aspekt ex-vivo unter Simulation physiologischer Bedingungen gehaltene, vitale Mammalia-Herzgewebe-Zellen. In einer bevorzugten Ausführungsform dieses Aspekts betrifft die Erfindung ex-vivo gehaltene, vitale Mammalia-Herzgewebe-Zellen in üblichem Kulturmedium unter Beaufschlagung mit Kulturgas bei physiologisch annehmbarem pH-Wert.

Weitere bevorzugte Ausführungsformen des ersten Aspekts der Erfindung ergeben sich aus den Unteransprüchen 2 bis 8.

Die Erfindung betrifft in einem zweiten Aspekt ein Verfahren zur Gewinnung von ex-vivo gehaltenen, vitalen Mammalia-Herzgewebe-Zellen, das die Schritte umfaßt:
- Gewinnen von Herzgewebe vom lebenden Mammalia-Herzen;
- Präparieren des gewonnenen Herzgewebes auf üblichem Weg unter Kühlen in einem an sich üblichen Präparationsmedium;
- zweidimensionales Schneiden der Herzgewebe-Stücke unter Erhalt zusammenhängender Herzgewebe-Schnitte;
- Teilen der Herzgewebe-Schnitte mittels einer Glaspipette unter Erhalt von Herzgewebe-Feinschnitten;
- Auswählen gleichförmiger intakter Herzgewebe-Feinschnitte; und
- Eintauchen und gegebenenfalls Belassen des/der Herzgewebe-Feinschnitte(s) in ein/einem geeignetes/geeigneten übliches/üblichen Kulturmedium unter Begasung mit Kulturgas bei einem physiologisch annehmbaren pH-Wert.

Eine alternative Ausführungsform der Erfindung gemäß diesem zweiten Aspekt betrifft ein Verfahren zur Gewinnung von ex-vivo gehaltenen, vitalen Mammalia-Herzgewebe-Zellen, das die Schritte umfaßt:
- Präparieren von vorher von Herzgewebe vom lebenden Mammalia-Herzen gewonnenem Herzgewebe auf üblichem Weg unter Kühlen in einem an sich üblichen Präparationsmedium;
- zweidimensionales Schneiden der Herzgewebe-Stücke unter Erhalt zusammenhängender Herzgewebe-Schnitte;
- Teilen der Herzgewebe-Schnitte mittels einer Glaspipette unter Erhalt von Herzgewebe-Feinschnitten;
- Auswählen gleichförmiger intakter Herzgewebe-Feinschnitte; und
- Eintauchen und gegebenenfalls Belassen des/der Herzgewebe-Feinschnitte(s) in ein/einem geeignetes/geeigneten übliches/üblichen Kulturmedium unter Begasung mit Kulturgas bei einem physiologisch annehmbaren pH-Wert.

Bevorzugte Ausführungsformen der Erfindung gemäß dem zweiten Aspekt ergeben sich aus den Unteransprüchen 11 bis 20.

In einem dritten Aspekt betrifft die vorliegende Erfindung ein Verfahren zum Kultivieren von ex-vivo gewonnenen, vitalen Mammalia-Herzgewebe-Zellen, das den Schritt umfaßt, daß man einen oder mehrere vom lebenden Mammalia-Herzen gewonnene(n) Herzgewebe-Feinschnitt(e) in ein geeignetes übliches Kulturmedium mit physiologisch annembarem pH-Wert unter Begasung mit Kulturgas eintaucht und gegebenenfalls diese(n) darin beläßt.

Bevorzugte Ausführungsformen der Erfindung gemäß dem dritten Aspekt ergeben sich aus den Unteransprüchen 22 bis 30.

Schließlich betrifft die Erfindung in einem vierten Aspekt die Verwendung der wie oben beschriebenen ex-vivo gehaltenen, vitalen Mammalia-Herzgewebe-Zellen in Modell-Untersuchungen chemischer, biologischer und/oder physikalischer Einflüsse auf physiologische oder pathophysiologischer Prozesse.

Bevorzugte Ausführungsformen der Erfindung gemäß dem vierten Aspekt ergeben sich aus den Unteransprüchen 32 bis 43.

Die erfindungsgemäßen Herzgewebe-Zellen sind von einem Säuger stammende Herzgewebe-Zellen. Säuger (Mammalia) im Sinne der vorliegenden Erfindung sind beliebige Säuger, wie beispielsweise Meerschweinchen, Mäuse, Hunde, Katzen oder Affen und sind in bevorzugten Ausführungsformen Menschen. Herzgewebe-Zellen vom Menschen sind deswegen besonders bevorzugt, weil sie für die denkbaren Verwendungen, wie sie nachfolgend im einzelnen beschrieben werden, die für das vitale Gewebemodell besten Ergebnisse liefern.

Die erfindungsgemäßen Mammalia-Herzgewebe-Zellen können ex-vivo gehalten werden, befinden sich also nicht mehr im Verband mit dem lebenden Mammalia-Herzen. Trotzdem sind die Zellen vital, zeigen also alle Funktionen des lebenden Gewebe-Verbandes. Insbesondere verhalten sich die erfindungsgemäßen Mammalia-Herzgewebe-Zellen wie Zellen im intakten Zellverband im lebenden Organ (Myokard), so dass funktionelle Untersuchungen der Interaktion der Zellen im Verband möglich werden.

Erfindungsgemäß werden die vitalen Mammalia-Herzgewebe-Zellen unter Simulation physiologischer Bedingungen gehalten. Darunter werden erfindungsgemäß im weitesten Sinn solche Bedingungen verstanden, die die Mammalia-Herzgewebe-Zellen benötigen, um ihre vitalen Funktionen beizubehalten. Es sind erfindungsgemäß solche Bedingungen anwendbar, die hinsichtlich des Aggregatzustandes, der Temperatur, der Zusammensetzung, des pH-Wertes, usw. des Mediums physiologische Bedingungen so weitgehend simulieren, daß die vitalen Funktionen der Mammalia-Herzgewebe-Zellen erhalten bleiben. Besonders bevorzugt sind Bedingungen in flüssiger Umgebung, d. h. Bedingungen des Haltens der erfindungsgemäßen Mammalia-Herzgewebe-Zellen in einem geeigneten flüssigen Kulturmedium. Dieses hat den Vorteil, daß die Bedingungen leicht und reproduzierbar eingestellt und aufrechterhalten werden können und das Medium einfach hinsichtlich seiner Zusammensetzung (gelöste Stoffe, pH-Wert, Gase) und seiner physikalischen Konditionen (Temperatur, Viskosität) flexibel auf geänderte Anforderungen reagieren kann.

In einer bevorzugten Ausführungform der Erfindung werden die Mammalia-Herzgewebe-Zellen in einem üblichen Kulturmedium gehalten. Als Kulturmedium kommt jegliches Kulturmedium infrage, das der Fachmann zur Aufbewahrung, Erhaltung der Funktionen von Zellen und Kultivierung von Zellen kennt. Auch Mischungen verschiedener Kulturmedien können verwendet werden. Mit Vorteil werden im wesentlichen auf Wasserbasis zusammengesetzte Kulturmedien verwendet, ohne daß die Erfindung hierauf beschränkt ist. Neben Wasser können auch andere flüssige Komponenten oder Lösungsmittel enthalten sein. Dem erfindungsgemäß verwendeten Kulturmedium bzw. der verwendeten Mischung kann/können ein oder mehrere Zusatz/Zusätze zugegeben sein, der/die eine Kultivierung der erfindungsgemäßen Zellen erlaubt/erlauben und/oder unterstützt/ unterstützen. Beispiele der Kulturmedien, die erfindungsgemäß einzeln oder in Kombination verwendet werden können, sind gewählt aus der Gruppe, die besteht aus Opti-MEM I serumreduziertes Medium, Iscove's modified Dulbecco's medium (IMDM), und Dulbecco's modified Eagle medium (D-MEM). Letzteres ist erfindungsgemäß besonders bevorzugt und stellt ein Kulturmedium dar, in dem die Kultur der erfindungsgemäßen ex-vivo gehaltenen, vitalen Mammalia-Herzgewebe-Zellen besonders gut gelingt. Übliche Zusätze sind gewählt aus Wachstumsfaktoren (z. B. Insulin, Transferrin), Pyruvat, Antioxidantien (z. B. Ascorbinsäure, Vitamin A, Vitamin E, Glutathion, Liponsäure), fetalem Kälberserum, Aminosäuren, Puffern zur Einstellung eines physiologisch annehmbaren pH-Wertes und anderen üblichen Zusätzen wie Lösungs- und Verdünnungsmitteln. Als ein besonders bevorzugtes Kulturmedium hat sich erfindungsgemäß Dulbecco's modified Eagle's medium (DMEM) + 20 % fetales Kälberserum + 2 mM L-Glutamin + 1 % non-essential amino acids (pH 7,4) herausgestellt. Die Komponenten dieses Kulturmediums sind im Handel von der Firma Gibco Invitrogen erhältlich. Die mit "non-essential amino acids" bezeichnete Mischung ist eine unter der genannten Bezeichnung von der Firma Gibco Invitrogen erhältliche Mischung der Aminosäuren L-Alanin, L-Asparagin, L-Aspagarinsäure, L-Glutaminsäure, Glycin, L-Prolin und L-Serin in den Mengen 890 mg/l, 1320 mg/l, 1330 mg/l, 1470 mg/l, 750 mg/l, 1150 mg/l bzw. 1050 mg/l.

Das Kulturmedium, in dem die erfindungsgemäßen ex-vivo gehaltenen, vitalen Mammalia-Herzgewebe-Zellen vorzugsweise gehalten werden, ist mit einem Kulturgas beaufschlagt. Das Kulturgas ist ein Gas, das der Kultivierung der Zellen dient und in dem Kulturmedium gelöst ist und/oder durch dieses hindurchstreicht, nachdem man es in das Kulturmedium eingeleitet hat. Denkbar ist auch, das eine zu dem Kulturgas abreagierbare oder das Kulturgas unter Kulturbedingungen freisetzende chemische Substanz in das Kulturmedium eingegeben wird. Aus ökonomischen Gründen wird regelmäßig ein Kulturgas in das Kulturmedium eingeleitet, wobei sich das Gas in dem flüssigen Medium zumindest teilweise löst; letzteres ist natürlich von den Kulturbedingungen (Temperatur, pH-Wert, Lösungsmittel etc.) abhängig. Bevorzugt wird als Kulturgas Luft verwendet. An diese müssen zur Vermeidung des Eintragens von die Kultivierung störenden Komponenten besondere Anforderungen gestellt werden. Besonders bevorzugt wird Reinluft oder sterile Luft verwendet, die auf dem Fachmann bekannten Wegen hergestellt bzw. bereitgestellt werden kann.

Ein wesentliches Merkmal des Kulturmediums ist der pH-Wert, der im physiologisch annehmbaren Bereich liegen muß. Dies bedeutet erfindungsgemäß, dass der pH-Wert in einem Bereich liegen muß, der die Kultivierung der erfindungsgemäßen Mammalia-Herzgewebe-Zellen nicht stört, sondern fördert. Erfindungsgemäß ist der physiologisch annehmbare pH-Wert vorzugsweise ein pH-Wert im Bereich von 6,5 bis 8, noch mehr vorzugsweise ein pH-Wert im Bereich von 6,9 bis 7,6, weiter bevorzugt ein pH-Wert im Bereich von 7,3 bis 7,5, noch weiter bevorzugt beispielsweise ein pH-Wert von 7,4.

Die Einstellung des pH-Wertes kann in an sich dem Fachmann bekannter Weise geschehen, beispielsweise durch Zugabe von chemischen Substanzen, die den pH-Wert auf den physiologisch annehmbaren Bereich einstellen. Bevorzugt ist die Einstellung des pH-Wertes über ein physiologisch annehmbares Puffersystem, was den zusätzlichen Vorteil bietet, dass bei Verschiebungen des pH-Wertes aufgrund chemischer Reaktionen der pH-Wert aufgrund der Gegenwart des Puffers konstant bleibt. Geeignete Puffer sind TRIS (Tris(hydroxymethyl-)aminomethan), HEPES (N-2-Hydroxyethylpiperazin-N'ethansulfonsäure), Phosphatpuffer, Citratpuffer oder Carbonat-Puffer. Erfindungsgemäß hat es sich als bevorzugt herausgestellt, das flüssige Kulturmedium über die Beaufschlagung mit dem Kulturgas (beispielsweise Reinluft) mit CO₂ zu puffern, vorzugsweise durch Beaufschlagung mit Reinluft mit einem erhöhten Anteil CO₂, verglichen mit dem natürlichen CO₂-Gehalt. Besonders bevorzugt enthält das Kulturgas Reinluft mit einem Anteil von 1 bis 5 Vol.-% CO₂, noch weiter bevorzugt Reinluft mit einem Anteil von 2 bis 3,5 Vol.-% CO₂, ganz besonders bevorzugt Reinluft mit einem Anteil von 3,3 Vol.-% CO₂. Alle vorgenannten Vol.-%-Angaben beziehen sich auf eine Temperatur der Kultivierung bzw. der Zufuhr des Kulturgases im Bereich der optimalen Temperatur für die Kultivierung der erfindungsgemäßen Zellen, beispielsweise eine Temperatur im Bereich von 32 bis 40 °C, vorzugsweise eine Temperatur im Bereich von 34 bis 38 °C, beispielsweise eine Temperatur von 36 °C.

Um die erfindungsgemäßen ex-vivo gehaltenen, vitalen Mammalia-Herzgewebe-Zellen optimal mit den in dem Kulturmedium enthaltenen Substanzen und dem Kulturgas versorgen zu können, ist es eine besonders vorteilhafte und damit bevorzugte Ausführungsform der Erfindung, dass sich die ex-vivo gehaltenen, vitalen Mammalia-Herzgewebe-Zellen auf einer für das Kulturmedium und/oder das Kulturgas durchlässigen Membran befinden. Diese kann - in für den Fachmann an sich bekannter Weise - derart ausgestaltet sein, dass handelsübliche Zell- und Gewebekultureinsätze, die in Gruppen auf Kulturplatten angeordnet sind, mit geeigneten natürlichen oder synthetischen oder natürlichen, aber synthetisch in geeigneter Weise modifizierten Membranen versehen sind, auf denen die erfindungsgemäßen ex-vivo gehaltenen, vitalen Mammalia-Herzgewebe-Zellen angeordnet sind, beispielsweise durch Aufbringen im Rahmen des Gewinnungsund/oder Kultivierungsverfahrens gemäß der Erfindung. Geeignete Membranen sind dem Fachmann bekannt. Es kommen beispielsweise synthetische Membranen mit der Handelsbezeichnung Anopore™ (Firma Nalge Nunc International) infrage, die einen Durchmesser von 25 mm und eine Porengröße von 0,02 µm haben. Ex-vivo gehaltene, vitale Mammalia-Herzgewebe-Zellen gemäß der Erfindung auf derartigen natürlichen oder synthetischen Membranen stehen in optimalem Austausch mit dem Kulturmedium und dem Kulturgas, so dass sie mit den für das Kultivieren nötigen Komponenten kontinuierlich versorgt werden. Das Ergebnis sind überraschen lange Zeiten, in denen eine ex-vivo-Kultivierung der Zellen erfolgen kann, ohne dass die natürlichen Funktionen verloren gehen. Letzteres ist die optimale Voraussetzung für eine erfolgreiche Untersuchung der Funktionen der erfindungsgemäßen Zellen bei den Verwendungen gemäß der Erfindung.

Besonders überraschend ist erfindungsgemäß, dass Mammalia-Herzgewebe-Zellen, insbesondere humane Herzgewebe-Zellen unter Aufrechterhaltung aller vitalen Funktionen, insbesondere aller herzspezifischen Funktionen der Zellen, gewonnen und kultiviert werden können. Bei Untersuchung der kultivierten Zellen nach der Entnahme aus dem lebenden Mammalia-Gewebe und Kultivierung gemäß dem nachfolgend beschriebenen Verfahren über 6 Tage zeigte sich eine suffiziente Vitalität der überwiegenden Mehrzahl der Zellen unter Erhalt der typischen Zellzusammensetzung und Gewebearchitektur.

Das erfindungsgemäße Verfahren zur Gewinnung von ex-vivo gehaltenen, vitalen Mammalia-Herzgewebe-Zellen umfasst als ersten Schritt die Gewinnung von Herzgewebe vom lebenden Mammalia-Herzen. In einer bevorzugten Ausführungsform wird Herzgewebe vom lebenden menschlichen Herzen gewonnen. Dies geschieht beispielsweise bei Gelegenheit von operativen Eingriffen am Herzen (bevorzugt am Human-Herzen), bei dem ein Anschluß an eine Herz-Lungen-Maschine erforderlich ist, ohne dass die Erfindung auf diese Ausführungsform beschränkt ist. Beispielsweise werden bei einem solchen Eingriff Teile des sog. Herzohrs entnommen. Es kann jedoch auch auf anderen - dem Fachmann bekannten - Wegen Mammalia-Herzgewebe vom lebenden Mammalia-Herzen entnommen werden.

Das so gewonnene Herzgewebe wird auf üblichem Weg unter Kühlen in einem an sich üblichen Präparationsmedium präpariert. Dies geschieht in dem Fachmann bekannter Weise, beispielsweise unter weitgehendem Entfernen von Fett- und Bindegewebe von dem vom lebenden Herzen entnommenen Mammalia-Herzgewebe-Stück unter Kühlung, so dass letztendlich vorzugsweise überwiegend Herzmuskelfaser-Gewebe herauspräpariert wird. Die Temperatur liegt in einem die Präparation erlaubenden, jedoch auch eine Verlangsamung physiologischer Reaktionen bewirkenden niedrigen Bereich, vorzugsweise im Bereich von 0 °C bis 6 °C, weiter bevorzugt im Bereich von 1 °C bis 5 °C, noch weiter bevorzugt im Bereich von 3 bis 4 °C.

Das bei der Überführung bzw. bei der Präparation der Gewebestücke zur Kühlung und Aufbewahrung beim Präparieren verwendete Präparationsmedium ist ein übliches, vom Fachmann bei solchen Schritten bekanntermaßen verwendeter Präparationsmedium. Dieses hat sinnvollerweise einen pH-Wert im physiologisch annehmbaren Bereich, vorzugsweise im Bereich von 6,5 bis 8, weiter bevorzugt im Bereich von 7,0 bis 7,6, noch weiter bevorzugt im Bereich von 7,2 bis 7,4, beispielsweise bei 7,35. In einer weiter bevorzugten Ausführungsform enthält es zusätzliche, für die Erhaltung der Gewebestücke vorteilhafte Substanzen, beispielsweise Wachstumsfaktoren, Insulin, Transferrin, Natriumpyruvat, B-27-Supplement (Firma Gibco Invitrogen), Folsäure, Vitamine, Seren (Pferd, Kalb, etc.), Kohlenhydrate, insbesondere Zucker (z. B. Glucose, Fructose, Sucrose, Trehalose etc.), Biotin, eine oder mehrere Aminosäure(n), beispielsweise L-Glutamin, einen oder mehrere Puffer, beispielsweise HEPES oder TRIS und gegebenenfalls auch gasförmige Komponenten bzw. Komponenten, die bei den vorherrschenden Bedingungen ein Gas oder Gase freisetzen können. Mit besonderem Vorteil wird als Präparationsmedium ein Medium der Zusammensetzung MEM-Hank's Medium unter Einschluß von 25 mM HEPES, 2 mM L-Glutamin (pH 7,35; bei 6 °C mit O₂ gesättigt) verwendet.

Der nächste Verfahrensschritt umfasst ein sog. zweidimensionales Schneiden der im vorangehenden Verfahrensschritt erhaltenen Herzgewebe-Stücke unter Erhalt zusammenhängender Herzgewebe-Schnitte. Dies erfolgt in einer bevorzugten Ausführungform des erfindungsgemäßen Verfahrens in zwei Schritten. Weiter bevorzugt ist eine zweischrittige Schneide-Verfahrensweise, bei der das zweidimensionale Schneiden der Herzgewebe-Stücke in der Weise erfolgt, dass man im ersten Schritt mit einer Schnittweite im Bereich von 0,3 bis 3 mm, vorzugsweise im Bereich von 0,8 bis 1,2 mm entgegen der Muskelfaser-Richtung schneidet und im zweiten Schritt die resultierenden Fragmente mit einer Schnittweite im Bereich von 100 bis 200 µm parallel zur Muskelfaser-Richtung schneidet. So lassen sich zusammenhängende Mammalia-Herzgewebe-Schnitte erhalten, die ex-vivo gehalten werden können und alle Eigenschaften lebenden Herzgewebes zeigen. Das zweidimensionale Schneiden kann mit Hilfe beliebiger Geräte erfolgen, die der Fachmann für derartige Zwecke zur Verfügung hat. Erfindungsgemäß wird bevorzugt ein Gerät mit der Bezeichnung McIllwain Chopper der Firma The Mickle Laboratory Engineering Co., Guildford, Surrey, Großbritannien verwendet. Die in diesem Verfahrensschritt erhaltenen, zum Teil noch zusammenhängenden Mammalia-Herzgewebe-Schnitte werden nachfolgend auf an sich bekannte Weise voneinander getrennt. Hierfür kann dem Fachmann für diesen Zweck vertrautes Equipment verwendet werden, beispielsweise Glaspipetten, Lanzetten oder ähnliches. Die Mammalia-Herzgewebe-Schnitte - wie auch die erhaltenen voneinander getrennten Mammalia-Herzgewebe-Feinschnitte - werden bei der vorgenannten niedrigen Temperatur, vorzugsweise in einem Präparationsmedium, weiter bevorzugt in einem kalten Präparationsmedium, besonders bevorzugt in dem bereits vorstehend genannten Präparationsmedium, das gekühlt wird, gehalten.

Von den so erhaltenen Mammalia-Herzgewebe-Feinschnitten werden diejenigen ausgewählt, die als Ergebnis des Gewinnungsverfahrens gleichförmig sind und intakt sind, d. h. vital sind und die vom lebenden Herzen bekannten Funktionen zeigen. Tests zur Ermittlung der Vitalität werden in den Beispielen weiter unten beschrieben.

Gemäß dem erfindungsgemäßen Verfahren werden die ausgewählten intakten Mammalia-Herzgewebe-Feinschnitte einzeln oder zu mehreren, bevorzugt einzeln, in ein übliches Kulturmedium eingegeben bzw. eingetaucht und gegebenenfalls in diesem belassen, wenn erforderlich über längere Zeit. Das Kulturmedium befindet sich bei einem physiologisch annehmbaren pH-Wert und wird mit einem Kulturgas begast.

In einer bevorzugten Ausführungsform des Verfahrens wird ein oder werden mehrere der geschnittenen und geteilten Mammalia-Herzgewebe-Schnitte vor dem oder während des Eintauchen(s) in das Kulturmedium auf eine für das Kulturmedium und das Kulturgas durchlässige Kulturmembran aufgegeben. Diese kann - in für den Fachmann an sich bekannter Weise - derart ausgestaltet sein, dass handelsübliche Zell- und Gewebekultureinsätze, die in Gruppen auf Kulturplatten angeordnet sind, mit geeigneten natürlichen oder synthetischen oder natürlichen, aber synthetisch in geeigneter Weise modifizierten Membranen versehen sind. Auf diesen wird eine oder werden mehrere der erfindungsgemäßen ex-vivo gehaltenen, vitalen Mammalia-Herzgewebe-Zellen angeordnet, beispielsweise durch Aufbringen vor dem letzten Schritt des Gewinnungsverfahrens gemäß der Erfindung. Geeignete Membranen sind dem Fachmann bekannt. Es kommen beispielsweise synthetische Membranen mit der Handelsbezeichnung Anopore^{TM} (Firma Nalge Nunc International) infrage, die einen Durchmesser von 25 mm und eine Porengröße von 0,02 µm haben. Ex-vivo gehaltene, vitale Mammalia-Herzgewebe-Zellen gemäß der Erfindung auf derartigen natürlichen oder synthetischen Membranen stehen in optimalem Austausch mit dem Kulturmedium und dem Kulturgas, so dass sie mit den für das Kultivieren nötigen Komponenten kontinuierlich versorgt werden.

Als Kulturmedium kommt jegliches Kulturmedium infrage, das der Fachmann zur Aufbewahrung, Erhaltung der Funktionen von Zellen und Kultivierung von Zellen kennt. Auch Mischungen verschiedener Kulturmedien können verwendet werden. Mit Vorteil werden im wesentlichen auf Wasserbasis zusammengesetzte Kulturmedien verwendet, ohne daß die Erfindung hierauf beschränkt ist. Neben Wasser können auch andere flüssige Komponenten oder Lösungsmittel enthalten sein. Dem in dem erfindungsgemäßen Verfahren verwendeten Kulturmedium bzw. der verwendeten Mischung kann/können ein oder mehrere Zusatz/Zusätze zugegeben sein, der/die eine Aufbewahrung und/oder Kultivierung der erfindungsgemäßen Zellen erlaubt/erlauben und/oder unterstützt/unterstützen. Beispiele der Kulturmedien, die erfindungsgemäß einzeln oder in Kombination verwendet werden können, sind gewählt aus der Gruppe, die besteht aus Opti-MEM I serumreduziertes Medium, Iscove's modified Dulbecco's medium (IMDM) und Dulbecco's modified Eagle medium (D-MEM). Letzteres ist erfindungsgemäß besonders bevorzugt und stellt ein Kulturmedium dar, in dem die Kultur und Aufbewahrung der erfindungsgemäßen ex-vivo gehaltenen, vitalen Mammalia-Herzgewebe-Zellen besonders gut gelingt. Übliche Zusätze sind gewählt aus Wachstumsfaktoren, Insulin, Transferrin, Natriumpyruvat, B-27-Supplement (Firma Gibco Invitrogen), Folsäure, Vitamine, Seren (Pferd, Kalb, etc., z. B. fetales Kälberserum), Kohlenhydrate, insbesondere Zucker (z. B. Glucose, Fructose, Sucrose, Trehalose etc.), Biotin, Aminosäuren, Puffern zur Einstellung eines physiologisch annehmbaren pH-Wertes und anderen üblichen Zusätzen wie Lösungs- und Verdünnungsmitteln. Als ein besonders bevorzugtes Kulturmedium hat sich erfindungsgemäß Dulbecco's modified Eagle's medium (DMEM) + 20 % fetales Kälberserum + 2 mM L-Glutamin + 1 % non-essential amino acids (pH 7,4) herausgestellt. Die Komponenten dieses Kulturmediums sind im Handel von der Firma Gibco Invitrogen erhältlich.

Erfindungsgemäß ist es außerdem bevorzugt, dass als physiologisch annehmbarer pH-Wert des Kulturmediums ein pH-Wert im Bereich von 6,5 bis 8 eingestellt wird, vorzugsweise ein pH-Wert im Bereich von 6,9 bis 7,6 eingestellt wird, weiter bevorzugt ein pH-Wert im Bereich von 7,3 bis 7,5 eingestellt wird, noch weiter bevorzugt ein pH-Wert von 7,4 eingestellt wird. Die Einstellung des pH-Werts erfolgt in der oben bereits im Detail beschriebenen Weise.

Erfindungsgemäß kann das Begasen des Kulturmediums mit jedem denkbaren Gas erfolgen, das die Vitalität der erfindungsgemäß erhaltenen Mammalia-Herzgewebe-Zellen erhält. Bevorzugt wird als Kulturgas Luft verwendet, die weiter bevorzugt Reinluft oder sogar sterile Luft ist. In einer weiter bevorzugten Ausführungsform des Verfahrens wird als Kulturgas Reinluft mit einem über dem natürlichen CO₂-Gehalt liegenden CO₂-Gehalt verwendet, weiter bevorzugt Reinluft mit einem CO₂-Gehalt von 1 bis 5 Vol.-%, noch mehr bevorzugt mit einem CO₂-Gehalt von 2 bis 3,5 Vol.-%, beispielsweise Reinluft mit einem CO₂-Gehalt von 3,3 Vol.-%. Ohne an diese Erklärung gebunden zu sein, wird angenommen, dass in der besonders bevorzugten Ausführungsform der Verwendung von mit CO₂ angereichertem Kulturgas das CO₂ mit dem Kulturmedium ein Lösungsgleichgewicht eingeht und in Form des H₂CO₃/HCO₃⁻-Puffersystems zur verbesserten Pufferung des vorgenannten Kulturmediums beiträgt.

Die vorliegende Erfindung betrifft auch ein Verfahren zum Kultivieren von ex-vivo gewonnenen, vitalen Mammalia-Herzgewebe-Zellen. Das Kultivier-Verfahren umfaßt den Schritt, daß man einen oder mehrere vom lebenden Mammalia-Herzen gewonnene(n) Herzgewebe-Feinschnitt(e) in ein geeignetes übliches Kulturmedium mit physiologisch annehmbarem pH-Wert unter Begasung mit Kulturgas eintaucht und gegebenenfalls diese(n) darin beläßt.

Gemäß bevorzugten Ausführungsformen des erfindungsgemäßen Kultivier-Verfahrens kann der/können die Mammalia-Herzgewebe-Feinschnitte vor dem Eintauchen in das Kulturmedium auf eine für das Kulturmedium und das Kulturgas durchlässige Membran aufgebracht werden. Pro Membran kann ein oder können mehrere Mammalia-Herzgewebe-Feinschnitt(e) aufgebracht werden. Für die Auswahl dem Membran und deren Herkunft können in weiter bevorzugten Ausführungsformen des Kultivierverfahrens dieselben Kriterien angewandt werden, wie sie oben im Zusammenhang mit dem Verfahren zur Gewinnung der erfindungsgemäßen Mammalia-Herzgewebe-Zellen beschrieben wurden. Gleiches gilt für die verwendbaren Kulturmedien und deren bevorzugte Zusammensetzung: Auch diese wurden oben im Zusammenhang mit dem letzten Schritt des Verfahrens zur Gewinnung der Mammalia-Herzgewebe-Zellen im einzelnen beschrieben, und die dortige Beschreibung der allgemeinen und bevorzugten Ausführungformen gilt sinngemäß auch für das vorliegende Kultivierverfahren.

In weiteren bevorzugten Ausführungsformen kann in dem vorliegenden Kultivierverfahren als physiologisch annehmbarer pH-Wert ein pH-Wert eingestellt werden, der im Bereich von 6,5 bis 8 liegt, vorzugsweise ein pH-Wert im Bereich von 6,9 bis 7,6, weiter bevorzugt ein pH-Wert im Bereich von 7,3 bis 7,5, beispielsweise ein pH-Wert von 7,4.

Die Begasung mit Kulturgas kann mit jedem geeigneten Gas erfolgen, das die Kultivierung der Mammalia-Herzgewebe-Zellen unterstützt. Weiter bevorzugt ist ein Kultivierverfahren, in dem die Begasung mit Reinluft erfolgt, noch mehr bevorzugt mit Reinluft mit einem gegenüber dem natürlichen CO₂-Gehalt erhöhten CO₂-Gehalt, weiter bevorzugt mit Reinluft mit 1 bis 5 Vol.-% CO₂, am meisten bevorzugt mit Reinluft mit 2 bis 3,5 Vol.-% CO₂, beispielsweise mit Reinluft mit 3,3 Vol.-% CO₂. Die Temperatur des Kulturmediums liegt dabei vorzugsweise bei 34 bis 38 °C, weiter bevorzugt bei 36 bis 37 °C.

Sowohl im Rahmen des oben beschriebenen Verfahrens zur Gewinnung der erfindungsgemäßen Mammalia-Herzgewebe-Zellen als auch im Rahmen des zuletzt beschriebenen Verfahrens zur Kultivierung solcher Mammalia-Herzgewebe-Zellen werden unter Aufrechterhaltung vitaler Funktionen, bevorzugt herzspezifischer Funktionen, Mammalia-Herzgewebe-Zellen erhalten, die ex-vivo gehalten werden können. Die Zeiten der vollständigen Aufrechterhaltung aller spezifischen Funktionen schwanken mit dem lebenden Ausgangsmaterial, den Bedingungen der Gewinnung und den verwendeten Kulturmedien und Kulturgasen. Sie legen in vorteilhaften Ausführungsformen bei bis zu 7 Tagen und mehr, wie sich aus den Beispielen ergibt.

Weitere bevorzugte Ausführungsformen der erfindungsgemäßen Verfahren umfassen die gemeinsame Kultivierung von ex-vivo gehaltenen, vitalen Mammalia-Herzgewebe-Zellen gemäß der Erfindung mit Zellen und/oder Gewebestücken anderen Ursprungs. Dabei ist die gleichzeitige Kultivierung der verschiedenen Zellen besonders bevorzugt. Die mit den erfindungsgemäßen Mammalia-Herzgewebe-Zellen kultivierten Zellen und/oder Gewebestücke anderen Ursprungs können beliebige Zellen bzw. Gewebestükke sein. Besonders bevorzugt sind andere Mammalia-Zellen und/oder -Gewebestücke, weiter bevorzugt andere Human-Zellen und/oder Human-Gewebestücke, als Herzgewebe-Zellen oder -Gewebestücke. Ebenfalls verwendbar für die gemeinsame, vorzugsweise gleichzeitige Kultivierung mit den erfindungsgemäßen Mammalia-Herzgewebe-Zellen sind Fremd-Zellen und/oder Fremd-Gewebestücke, weiter bevorzugt Zellen aus der Gruppe Bakterien-Zellen, Virus-Zellen oder Pilz-Zellen, ohne die Erfindung in dieser Ausführungsform hierauf zu beschränken.

Die Erfindung betrifft auch die Verwendung der erfindungsgemäßen, ex-vivo gehaltenen, vitalen Mammalia-Herzgewebe-Zellen gemäß der obigen Beschreibung, insbesondere die Verwendung der nach dem erfindungsgemäßen Verfahren erhaltenen Mammalia-Herzgewebe-Zellen, in Modell-Untersuchungen chemischer, biologischer und/oder physikalischer Einflüsse auf physiologische oder pathophysiologische Prozesse, besonders bevorzugt derartiger Prozesse des Mammalia-Herzens, und ganz besonders bevorzugt des menschlichen Herzens.

Durch die Bereitstellung der Mammalia-Herzgewebe-Zellen werden erstmals systematische funktionelle Untersuchungen am humanen und tierischen Vorhof-Myokard möglich. So können unter anderem der Effekt von Rezeptor-Agonisten und -Antagonisten auf die myokardiale Signaltransduktion mit molekularbiologischen und elektrophysiologischen Methoden untersucht werden.

Die in Kultur gehaltenen Gewebeschnitte können über Kohlefaserelektroden auch elektrisch über eine "elektrische Feldstimulation" erregt werden. Hierdurch können durch die Variation der angelegten Spannung, der Elektroden bzw. der Stimuluskonfiguration, der Frequenz der applizierten Impulse bzw. deren Rhythmik Herzrhythmusstörungen simuliert werden. Die Auswirkungen auf das Gewebe können nachfolgend durch pathologische (Histologie, Immunhistochemie etc.), elektrophysiologische (Mikroelektrodentechnik, Patch-Clamp etc.) und molekulare Untersuchungsmethoden (Western Blotting, PCR etc.) analysiert werden.

Für Vorhofgewebe erscheint in diesem Zusammenhang gerade die Untersuchung während bzw. nach schneller elektrischer Stimulation von Bedeutung, da durch diese Stimulationsform die Veränderungen des Herzvorhofgewebes bei Patienten mit tachykarden Vorhofrhythmus-Störungen (vor allem des Vorhofflimmerns) reproduziert werden können. Weil die Wand der Herzvorhöfe sehr dünn ist, kann eine sequentielle Biopsie der Vorhöfe in vivo nicht erfolgen. Das durch die Erfindung bereitgestellte Modell der Kultivierung von Vorhofgewebe mit begleitender elektrischen Stimulation erlaubt somit erstmalig funktionelle Analysen im intaktem Gewebeverband.

Die erfindungsgemäßen Untersuchungen haben gezeigt, dass die schnelle elektrische Stimulation von Vorhofgewebe innerhalb von 24 Stunden zu gleichen zellulären Veränderungen der Genexpression führt, wie sie bei Patienten mit Vorhofflimmern nachweisbar sind. So finden sich eine gesteigerte Expression des "angiotensin-converting enzyms" (ACE), Regulation der Angiotensin II-Rezeptoren sowie Aktivierung von "mitogen-activated protein kinase" (MAP-Kinase Erk2) (siehe nachfolgendes Beispiel 2), wie sie auch schon bei Patienten mit Vorhofflimmern nachgewiesen worden sind.

In Analogie zum Vorhofflimmern können in ähnlicher Weise auch die Effekte von tachykarden Rhythmusstörungen im Gewebe der Herzkammern untersucht werden. Hierbei ist auch die Untersuchung von hohen elektrischen Feldstärken (Simulation von Defibrillation bzw. Kardioversion) auf das Myokard von grossem Interesse.

Eine elektrische Stimulation von ex-vivo kultivierten Gewebeverbänden erfolgt beispielsweise über Kohleelektroden mittels elektrischer Feldstimulation. Die Kohleelektroden werden über Platindrähte mit einem handelsüblichen Stimulator konnektiert. Zur Stimulation werden monophasische oder biphasische Gleistrom-Stimuli mit ca. 150 V im Medium appliziert, wobei die in Kultur gehaltenen Mammalia-Herzgewebe-Zellen im Zentrum des elektrischen Feldes positioniert werden. Die Frequenz der Stimulation sowie die Stimulusdauer können entsprechend der elektrophysiologischen Notwendigkeit variiert werden, um eine suffiziente elektrische Erregung des Gewebes zu gewährleisten. Die Reizantwort des Gewebes kann über Mikoelektrodensysteme bzw. auch durch Auflichtmikroskopie kontrolliert werden. Die Stimulationselektroden werden zur längerdauerenden Stimulation über Verlängerungskabel bis in den Zellkulturschrank geleitet, wodurch eine mehrtägige Stimulation unter optimalen Temperaturbedingungen möglich ist.

Im einzelnen können die erfindungsgemäßen ex-vivo gehaltenen, vitalen Mammalia-Herzgewebe-Zellen in folgenden Bereichen verwendet werden:
- Definierte exogene Stimuli, die chemische, biologische und physikalische Reize umfassen, wirken auf das Herzgewebe ein, und die Wirkungen dieser Reize auf Morphologie und Funktion des Herzgewebes und dessen Bestandteile werden bestimmt.
- Die Mammalia-Herzgewebe-Zellen werden verwendet zur Target-Identifizierung und Target-Validierung, zur Identifizierung und Validierung von diagnostischen Markern und zur Entwicklung diagnostischer Werkzeuge zur Früherkennung bzw. Akutdiagnose von kardiovaskulären Erkrankungen.
- Die Mammalia-Herzgewebe-Zellen werden verwendet zur Aufklärung von physiologischen, vorzugsweise pathophysiologischen Wirkungsmechanismen bei kardiovaskulären Erkrankungen, besonders bevorzugt bei Herzrhythmusstörungen, bei ischämischen Erkrankungen und bei der Aufklärung von Präconditioning-Effekten für die Arzneimittelentwicklung.
- Die Mammalia-Herzgewebe-Zellen werden verwendet zum Wirkstoffscreening und zur Wirkstoffidentifizierung und Validierung einschließlich der Verwendung als Toxizitätsassay.
- Die Mammalia-Herzgewebe-Zellen werden verwendet zur Entwicklung von Arzneimitteln für die Behandlung von Erkrankungen des kardiovaskulären Systems.
- Durch biologisch oder pharmakologisch aktive Substanzen oder Substanzen werden chemische Reize ausgeübt, die zum Test und zur Entwicklung präventiver oder therapeutisch relevanter Substanzen dienen.
- Durch Bakterien, Viren, Pilze oder Einzeller bzw. deren Bestandteile wie Haptene, Antikörper oder Antigene tierischen bzw. menschlichen Ursprungs, Peptide, Proteine, DNS, RNS oder andere Makromoleküle werden (mikro-) biologische Reize ausgeübt, die zelluläre Funktionen beeinflussen können.
- Durch elektromagnetische oder radioaktive Strahlung, elektrische Stimulation, mechanische Reize (vorzugsweise Dehnung), Veränderungen der Temperatur, des Druckes oder des Sauerstoff- und Kohlendioxid-Gehaltes der Luft oder des Kulturmediums werden physikalische Reize ausgeübt.
- Funktionen des Mammalia-Herzgewebes, vorzugsweise des menschlichen Herzgewebes, ist/sind die zelluläre Vitalität, die gewebespezifische Genexpression aufmRNS- und Protein-Ebene, die Ionenhomöostase, der Metabolismus, die Signaltransduktion, die Fähigkeit zur Regeneration bzw. Teilung von dazu befähigten Zellen, die elektrische Erregbarkeit, die elektrische Leitfähigkeit und/oder die Kontraktilität.
- Die Morphologie des Herzgewebes ist/sind die Anzahl, relative Häufigkeit, Lokalisation, Anordnung, Form und/oder Größe aller im Gewebe befindlichen Zellen und Zelltypen, vorzugsweise der Myozyten, Fibroblasten, Leukozyten, Nervenzellen und Endothelzellen.
- Die Morphologie des Herzgewebes ist/sind die subzelluläre Charakteristik der Zelltypen, vorzugsweise die Zahl und Größe von Mitochondrien, anderen Zellorganellen, und/oder die Integrität des kontraktilen Apparates bzw. Zytoskeletts.

Die Erfindung wird durch die nachfolgenden Beispiele näher erläutert, ohne auf die Beispiele beschränkt zu sein.

### Beispiel 1

### Gewinnung und Untersuchung der Vitalität von humanen Herzgewebe-Zellen (Vorhof-Schnitten)

Für die Präparation der Gewebeschnitte wurde humanes Herzgewebe verwendet. Dabei handelte es sich um Teile des Herzohrs.

Das Gewebe wurde im Rahmen eines operativen Eingriffs am Herzen entfernt, bei dem der Anschluß einer Herz-Lungen-Maschine notwendig war. Der Transfer bzw. die Präparation der Gewebeslices erfolgte eisgekühlt, bei ca. 4°C in einem Präparationsmedium der folgenden Zusammensetzung: MEM-Hanks' Medium plus 25 mM HEPES, 2 mM L-Glutamin (pH 7.35 bei 6 °C, gesättigt mit O₂).

Von dem entnommenen Gewebestück wurde Fett- bzw. Bindegewebe weitgehend entfernt, so daß überwiegend Muskelfasergewebe übrig blieb. Die Gewebeschnitte wurden durch zweidimensionales Schneiden mit Hilfe eines McIllwain Choppers (The Mickle Laboratory Engineering Co., Guildford, Surrey, UK) erhalten. Im ersten Schritt erfolgte ein Schneiden mit einer Schnittweite von 1 mm entgegen der Faserrichtung und im zweiten Schritt wurden dann die resultierenden Fragmente mit einer Schnittweite von 150 µm parallel zur Faserrichtung geteilt.

In eiskaltem Präparationspuffer erfolgte dann das Teilen noch zusammenhängender Gewebestücke mittels einer Glaspipette.

Für die Kultivierung wurden intakte, gleichförmige Gewebeschnitte ausgewählt. Diese wurden auf Anopore^{TM}-Membranen (Ø 25 mm, Porengröße 0.02 µm) von Zell- und Gewebekultureinsätzen (Firma Nunc, Wiesbaden) überführt. In der Regel wurden dabei ca. 5 bis 10 Schnitte/Membran kultiviert. Nach ihrer Beschickung wurden die Einsätze in Nunclon^{TM}-Zell- und Gewebekultur-6-Lochplatten (Firma Nunc, Wiesbaden) gesetzt. Diese enthielten 1.2 ml Kulturmedium/Loch. Das Kulturmedium setzte sich wie folgt zusammen: Dulbecco's modified Eagle's medium (DMEM) plus 20 % fetales Kälberserum, 2 mM L-Glutamin, 1 % non essential amino acids (pH 7.4) (alle Artikel von Firma Gibco Invitrogen). Während der Kultivierung wurden die Kulturen mit 3,3 % CO₂ bei 36°C begast. Dreimal pro Woche wurde ein Mediumwechsel durchgeführt.

Nach 6 Tagen Kultivierung erfolgten die Untersuchungen zur Vitalität der kultivierten Explantate.

Die Zellen wurden mit Propidiumiodid (rot) zur Darstellung toter Zellkerne sowie mit SYTO13 (grün) (Firma Molecular Probes, Eugene, OR, USA) zur Darstellung vitaler Zellkerne gefärbt, wie beschrieben wurde von Lendeckel et al., Journal of Ethnopharmacology 79 (2002) 221-227.

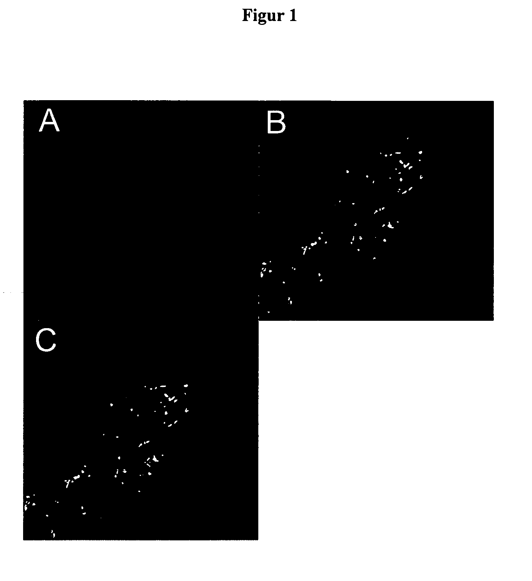

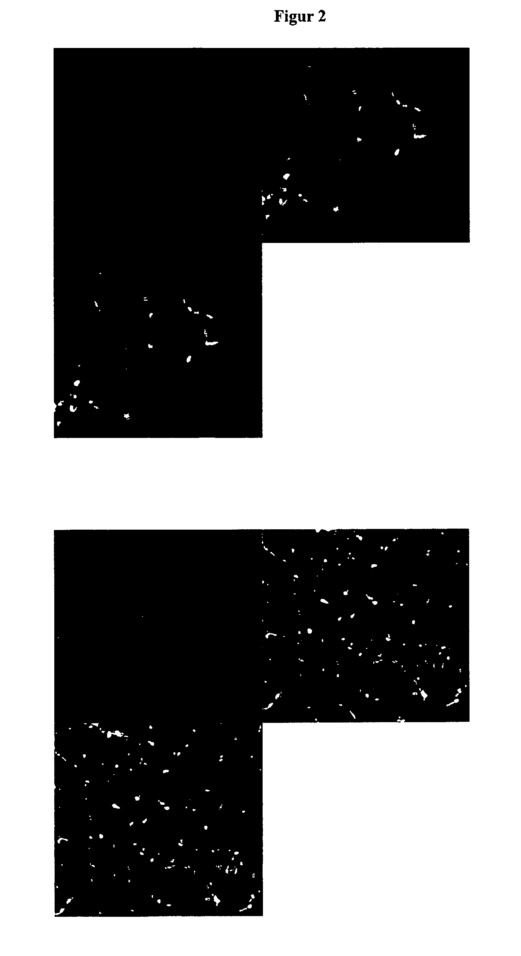

Die Ergebnisse sind in den Figuren 1 und 2 dargestellt. Dabei ist Figur 1A eine Darstellung der Rot-Fluoreszenz; ist Figur 1B eine Darstellung der Grün-Fluoreszenz; und ist Figur 1C die Überlagerung von Figur 1A und Figur 1B. Figur 2 zeigt die Ergebnisse der Untersuchung der Vitalität von humanen Vorhof-Schnitten nach 8-tägiger Kultivierung in-vitro. Die Färbung ist die gleiche wie vorstehend zu Figur 1 angegeben; Figur 2 zeigt jedoch eine stärkere Vergrößerung. Dabei sind Figur 2A und Figur 2D die Darstellungen der Färbung mit Propidiumiodid; sind Figur 2B und Figur 2E die Darstellungen der Färbung mit SYTO13; und sind Figur 2C und Figur 2F Überlagerungen der Figuren 2A+2B und der Figuren 2D+2E.

Figur 1 und Figur 2 zeigen, dass nach mehrtägiger Kultivierung *in-vitro* von humanen Vorhofschnitten die überwiegende Mehrzahl der Zellen vital ist und die typische Zellzusammensetzung und Gewebearchitektur erhalten bleibt.

### Beispiel 2

### Induktion der Expression von "angiotensin-converting enzyme" (ACE) sowie der MAP-Kinase Erk2 in in-vitro elektrisch stimulierten Herzvorhof-Gewebeschnitten.

Die gemäß Beispiel 1 erhaltenen Gewebeschnitte wurden über einen Zeitraum von 24 Stunden elektrisch stimuliert oder unter ansonsten identischen Bedingungen ohne elektrische Stimulation kultiviert. Die elektrische Stimulation erfolgte mit einer Spannung von 12 V/cm, und die Frequenz betrug 36 min⁻¹ (entspricht Sinusrhythmus) oder 120 min⁻¹ (entspricht Vorhof-Flimmem). Bei der Simulation des Vorhof-Flimmerns (120 min⁻¹) kommt es zu einer starken Induktion von ACE- und Erk2-mRNS gegenüber den nicht-stimulierten Kontrollen. Diese Veränderungen treten bei niedrigfrequenter Stimulation (Sinusrhythmus, SR) nicht auf. Vielmehr kommt es unter diesen Bedingungen eher zu einer Verminderung der ACE- und Erk2-Expression. Die gleichen Effekte hinsichtlich der Expression von ACE und Erk2 wurden bereits in ex vivo-Material von Patienten mit Vorhof-Flimmern im Vergleich zu Patienten mit SR beobachtet [Goette, A. et al.: Regulation of angiotensin II receptor subtypes during atrial fibrillation in humans; Circulation 101 (2000), 2678-2681; Goette, A. et al.: Increased Expression of extracellular signal-regulated kinase and angiotensin-converting enzyme in human atria during atrial fibrillation; Journal of the American College of Cardiology 35: 1669-1677, 2000].

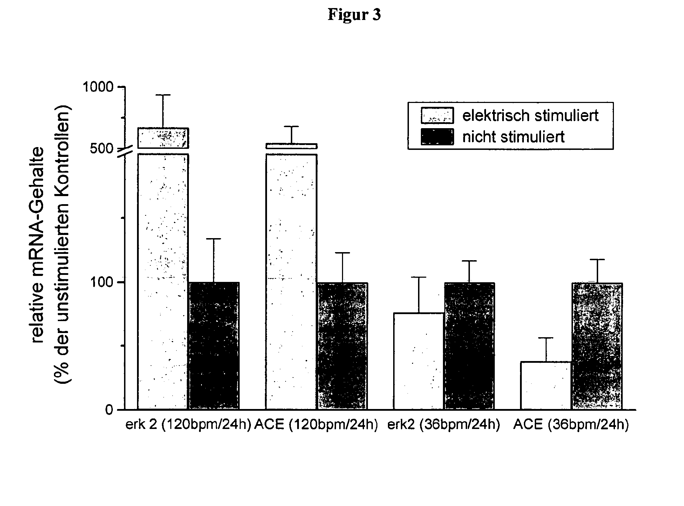

Figur 3 zeigt die mRNS-Expression von ACE und Erk2 in Herzgewebe-Schnitten nach Kultivierung *ex-vivo.*

### Beispiel 3

### Induktion der Expression des κ1-Opiatrezeptors (KOR) auf mRNS-Ebene nach elektrischer Stimulation von in vitro-differenzierten Kardiomyozyten (P19-Zellen) sowie in humanen Herzvorhof-Gewebeschnitten.

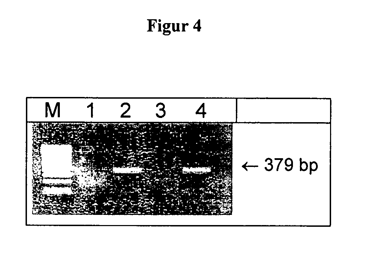

Aus den gemäß Beispiel 1 hergestellten Zellen bzw. Geweben wurde nach 24-stündiger elektrischer Stimulation die Gesamt-RNS mittels Standard-Verfahren isoliert. Der Nachweis von KOR-mRNS erfolgte mittels Polymerase-Ketten-Reaktion (PCR) und Darstellung der PCR-Produkte anhand der Agarose-Gel-Elektrophorese und Ethidiumbromid-Färbung. Figur 4 zeigt den Nachweis von KOR-mRNS in elektrisch stimulierten in vitro-differenzierten Kardiomyozyten (P19-Zellen) sowie in elektrisch stimulierten Human-Herzvorhof-Gewebeschnitten. Eine KOR-Expression wurde sowohl in P19-Zellen (Bahnen 1 und 2) als auch in Gewebeschnitten (Bahnen 3 und 4) beobachtet, jedoch ausschließlich nach vorangegangener elektrischer Stimulation (120 bpm) (Bahnen 2 und 4).

## Patentansprüche

1. *Ex-vivo* unter Simulation physiologischer Bedingungen gehaltene, vitale Mammalia-Herzgewebe-Zellen.

2. *Ex-vivo* gehaltene, vitale Mammalia-Herzgewebe-Zellen nach Anspruch 1 in üblichem Kulturmedium unter Beaufschlagung mit Kulturgas bei physiologisch annehmbarem pH-Wert.

3. Mammalia-Herzgewebe-Zellen nach Anspruch 1 oder Anspruch 2, worin der physiologisch annehmbare pH-Wert ein pH-Wert im Bereich von 6,5 bis 8 ist, vorzugsweise ein pH-Wert im Bereich von 6,9 bis 7,6 ist, weiter bevorzugt ein pH-Wert im Bereich von 7,3 bis 7,5 ist, noch weiter bevorzugt ein pH-Wert von 7,4 ist, und/oder worin das Kulturmedium Opti-MEM I serumreduziertes Medium, Iscove's modified Dulbecco's medium (IMDM) und Dulbecco's modified Eagle medium (D-MEM) ist, vorzugsweise Opti-MEM I serumreduziertes Medium + 2 mM Glutamin + 1 % non-essential amino acids + B27 supplement oder Dulbecco's modified Eagle medium (D-MEM) + 20 % fetales Kälberserum + 2 mM L-Glutamin + 1 % non-essential amino acids (pH 7,4) ist, und/oder worin das Kulturgas Reinluft ist, vorzugsweise Reinluft mit erhöhtem Anteil an CO₂ ist, weiter bevorzugt Reinluft mit 1 bis 5 Vol.-% CO₂ ist, noch weiter bevorzugt Reinluft mit 2 bis 3,5 % CO₂ ist.

4. Mammalia-Herzgewebe-Zellen nach einem der Ansprüche 1 bis 3 auf einer für das Kulturmedium und/oder das Kulturgas durchlässigen Membran.

5. Mammalia-Herzgewebe-Zellen nach einem der Ansprüche 1 bis 4 vom menschlichen Herzen.

6. Mammalia-Herzgewebe-Zellen nach einem der Ansprüche 1 bis 5 unter Aufrechterhaltung aller vitalen Funktionen, vorzugsweise aller herzspezifischen Funktionen der Zellen.

7. Verfahren zur Gewinnung von *ex-vivo* gehaltenen, vitalen Mammalia-Herzgewebe-Zellen, umfassend die Schritte:
- Gewinnen von Herzgewebe vom lebenden Mammalia-Herzen;
- Präparieren des gewonnenen Herzgewebes auf üblichem Weg unter Kühlen in einem an sich üblichen Präparationsmedium;
- zweidimensionales Schneiden der Herzgewebe-Stücke unter Erhalt zusammenhängender Herzgewebe-Schnitte;
- Teilen der Herzgewebe-Schnitte mittels einer Glaspipette unter Erhalt von Herzgewebe-Feinschnitten;
- Auswählen gleichförmiger intakter Herzgewebe-Feinschnitte; und
- Eintauchen und gegebenenfalls Belassen des/der Herzgewebe-Feinschnitte(s) in ein/einem geeignetes/geeigneten übliches/üblichen Kulturmedium unter Begasung mit Kulturgas bei einem physiologisch annehmbaren pH-Wert.

8. Verfahren zur Gewinnung von *ex-vivo* gehaltenen, vitalen Mammalia-Herzgewebe-Zellen, umfassend die Schritte:
- Präparieren von vorher von Herzgewebe vom lebenden Mammalia-Herzen gewonnenem Herzgewebe auf üblichem Weg unter Kühlen in einem an sich üblichen Präparationsmedium;
- zweidimensionales Schneiden der Herzgewebe-Stücke unter Erhalt zusammenhängender Herzgewebe-Schnitte;
- Teilen der Herzgewebe-Schnitte mittels einer Glaspipette unter Erhalt von Herzgewebe-Feinschnitten;
- Auswählen gleichförmiger intakter Herzgewebe-Feinschnitte; und
- Eintauchen und gegebenenfalls Belassen des/der Herzgewebe-Feinschnitte(s) in ein/einem geeignetes/geeigneten übliches/üblichen Kulturmedium unter Begasung mit Kulturgas bei einem physiologisch annehmbaren pH-Wert.

9. Verfahren nach einem der Ansprüche 7 oder 8, worin als Mammalia-Herzgewebe menschliches Herzgewebe verwendet wird und/oder worin überwiegend Herzmuskelfaser-Gewebe verwendet wird.

10. Verfahren nach einem der Ansprüche 7 bis 9, worin als Präparationsmedium ein übliches Präparationsmedium mit einem pH-Wert im physiologisch annehmbaren Bereich verwendet wird, vorzugsweise worin MEM-Hank's Medium unter Einschluß von 25 mM HEPES, 2 mM L-Glutamin (pH 7,35; bei 6 °C mit O₂ gesättigt) verwendet wird, und/oder worin als Temperatur des Kühlschrittes eine Temperatur im Bereich von 0 bis 6 °C eingestellt wird, vorzugsweise eine Temperatur im Bereich von 1 bis 5 °C, noch weiter bevorzugt eine Temperatur im Bereich von 3 bis 4 °C eingestellt wird und/oder worin als physiologisch annehmbarer pH-Wert ein pH-Wert im Bereich von 6,5 bis 8 eingestellt wird, vorzugsweise ein pH-Wert im Bereich von 6,9 bis 7,6 eingestellt wird, weiter bevorzugt ein pH-Wert im Bereich von 7,3 bis 7,5 eingestellt wird, noch weiter bevorzugt ein pH-Wert von 7,4 eingestellt wird.

11. Verfahren nach einem der Ansprüche 7 bis 10, worin das zweidimensionale Schneiden der Herzgewebe-Schnitte in zwei Schritten erfolgt, vorzugsweise worin das zweidimensionale Schneiden der Herzgewebe-Stücke in der Weise erfolgt, daß man im ersten Schritt mit einer Schnittweite im Bereich von 0,3 bis 3 mm, vorzugsweise im Bereich von 0,8 bis 1,2 mm entgegen der Muskelfaser-Richtung schneidet und im zweiten Schritt die resultierenden Fragmente mit einer Schnittweite im Bereich von 100 bis 200 µm parallel zur Muskelfaser-Richtung schneidet.

12. Verfahren nach einem der Ansprüche 7 bis 11, worin das Teilen der Herzgewebe-Stücke mit einer Glaspipette in einem Präparationsmedium erfolgt, bevorzugt in einem kalten Präparationsmedium, weiter bevorzugt in einem kalten Präparationsmedium wie in Anspruch 11 beansprucht.

13. Verfahren nach einem der Ansprüche 7 bis 12, worin die geschnittenen und geteilten Herzgewebe-Schnitte vor dem oder während des Eintauchen(s) in das Kulturmedium auf eine für das Kulturmedium und das Kulturgas durchlässige Kulturmembran aufgegeben werden.

14. Verfahren nach einem der Ansprüche 7 bis 13, worin die Gewebeschnitte mit Reinluft begast werden, vorzugsweise mit Reinluft mit 1 bis 5 Vol.-% CO₂ begast werden, weiter bevorzugt mit Reinluft mit 2 bis 3,5 Vol.-% CO₂ begast werden.

15. Verfahren zum Kultivieren von *ex-vivo* gewonnenen, vitalen Mammalia-Herzgewebe-Zellen, umfassend den Schritt, daß man einen oder mehrere vom lebenden Mammalia-Herzen gewonnene(n) Herzgewebe-Feinschnitt(e) in ein geeignetes übliches Kulturmedium mit physiologisch annehmbarem pH-Wert unter Begasung mit Kulturgas eintaucht und gegebenenfalls diese(n) darin beläßt.

16. Verfahren nach Anspruch 15, worin man den/die Herzgewebe-Feinschnitt(e) vor dem oder während des Eintauchen(s) in das Kulturmedium auf eine für das Kulturmedium und das Kulturgas durchlässige Kulturmembran aufgibt.

17. Verfahren nach Anspruch 15 oder Anspruch 16, worin man als Mammalia-Herzgewebe-Zellen menschliche Herzgewebe-Zellen verwendet.

18. Verfahren nach einem der Ansprüche 15 bis 17, worin die Kultivierung in Opti-MEM I serumreduziertem Medium + 2 mM Glutamin + 1 % non-essential amino acids + B27 supplement oder in Dulbecco's modified Eagle medium (D-MDM) + 20 % fetales Kälberserum + 2 mM L-Glutamin + 1 % non-essential amino acids (pH 7,4) erfolgt und/oder worin als physiologisch annehmbarer pH-Wert ein pH-Wert im Bereich von 6,5 bis 8 eingestellt wird, vorzugsweise ein pH-Wert im Bereich von 6,9 bis 7,6 eingestellt wird, weiter bevorzugt ein pH-Wert im Bereich von 7,3 bis 7,5 eingestellt wird, noch weiter bevorzugt ein pH-Wert von 7,4 eingestellt wird und/oder worin die Temperatur des Kulturmediums bei 34 bis 38 °C liegt, vorzugsweise bei 36 bis 37 °C liegt.

19. Verfahren nach einem der Ansprüche 21 bis 24, worin die Gewebeschnitte mit Reinluft begast werden, vorzugsweise mit Reinluft mit 1 bis 5 Vol.-% CO₂ begast werden, weiter bevorzugt mit Reinluft mit 2 bis 3,5 Vol.-% CO₂ begast werden.

20. Verfahren nach einem der Ansprüche 7 bis 19 unter Aufrechterhaltung vitaler, bevorzugt herzspezifischer Funktionen des Herzmuskel-Gewebeverbandes.

21. Verfahren nach einem der Ansprüche 7 bis 20, umfassend die gemeinsame, vorzugsweise die gleichzeitige Kultivierung von Mammalia-Herzgewebe-Zellen mit Zellen und/oder Gewebestücken anderen Ursprungs, vorzugsweise worin die Zellen oder Gewebestücke anderen Ursprungs Mammalia-Zellen und/oder -Gewebestücke, weiter vorzugsweise Human-Zellen und/oder -Gewebestücke, und/oder Fremd-Zellen und/oder -Gewebestücke, weiter vorzugsweise Zellen aus der Gruppe Bakterien-Zellen, Virus-Zellen und Pilz-Zellen, sind.

22. Verwendung ex-vivo gehaltener, vitaler Mammalia-Herzgewebe-Zellen nach einem der Ansprüche 1 bis 6 in Modell-Untersuchungen chemischer, biologischer und/oder physikalischer Einflüsse auf physiologische oder pathophysiologischer Prozesse vorzugsweise in Modell-Untersuchungen chemischer, biologischer und/oder physikalischer Einflüsse auf physiologische oder pathophysiologischer Prozesse des Mammalia-Herzens, weiter vorzugsweise des menschlichen Herzens.

23. Verwendung nach Anspruch 22,
- worin definierte exogene Stimuli, umfassend chemische, biologische und physikalische Reize, auf das Herzgewebe einwirken und die Wirkungen dieser Reize auf Morphologie und Funktion des Herzgewebes und dessen Bestandteile bestimmt werden und/oder
- zur Target-Identifizierung und Target-Validierung, zur Identifizierung und Validierung von diagnostischen Markern und zur Entwicklung diagnostischer Werkzeuge zur Früherkennung bzw. Akutdiagnose von kardiovaskulären Erkrankungen und/oder
- zur Aufklärung von physiologischen, vorzugsweise pathophysiologischen Wirkungsmechanismen bei kardiovaskulären Erkrankungen, besonders bevorzugt bei ischämischen Erkrankungen und bei der Aufklärung von Präconditioning-Effekten für die Arzeneimittelentwicklung und/oder
- zum Wirkstoffscreening und zur Wirkstoffidentifizierung und Validierung einschließlich der Verwendung als Toxizitätsassay und/oder
- zur Arzeneimittelentwicklung für die Behandlung von Erkrankungen des kardiovaskulären Systems und/oder
- worin durch biologisch oder pharmakologisch aktive Substanzen oder Substanzen chemische Reize ausgeübt werden, die zur Testung und Entwicklung präventiver oder therapeutisch relevanter Substanzen dienen und/oder
- worin durch Bakterien, Viren, Pilze oder Einzeller bzw. deren Bestandteile wie Haptene, Antikörper oder Antigene tierischen bzw. menschlichen Ursprungs, Peptide, Proteine, DNS, RNS oder andere Makromoleküle (mikro-) biologische Reize ausgeübt werden, die zelluläre Funktionen beeinflussen können und/oder
- worin durch elektromagnetische oder radioaktive Strahlung, elektrische Stimulation, mechanische Reize (vorzugsweise Dehnung), Veränderungen der Temperatur, des Druckes oder des Sauerstoff- und Kohlendioxid-Gehaltes der Luft oder des Kulturmediums physikalische Reize ausgeübt werden und/oder
- worin Funktionen des Mammalia-Herzgewebes, vorzugsweise des menschlichen Herzgewebes, die zelluläre Vitalität, die gewebespezifische Genexpression auf mRNS- und Protein-Ebene, die Ionenhomöostase, der Metabolismus, die Signaltransduktion, die Fähigkeit zur Regeneration bzw. Teilung von dazu befähigten Zellen, die elektrische Erregbarkeit, die elektrische Leitfähigkeit und/oder die Kontraktilität ist/sind und/oder
- worin die Morphologie des Herzgewebes die Anzahl, relative Häufigkeit, Lokalisation, Anordnung, Form und/oder Größe aller im Gewebe befindlichen Zellen und Zelltypen, vorzugsweise der Myozyten, Fibroblasten, Leukozyten, Nervenzellen und Endothelzellen, ist/sind und/oder
- worin die Morphologie des Herzgewebes die subzelluläre Charakteristik der Zelltypen, vorzugsweise die Zahl und Größe von Mitochondrien, anderen Zellorganellen, und/oder die Integrität des kontraktilen Apparates bzw. Zytoskeletts ist/sind.
